# EUROPEAN PATENT APPLICATION

(11) **EP 0 594 148 A1**
(43) Date of publication of application: **27.04.1994**
(21) Application number: 93116969.2
(22) Date of filing: 20.10.1993
(51) Int. Cl.: A61L 31/00

(54) **Bioabsorbable foam pledgets**

(30) Priority: 21.10.1992 US 964436
(71) Applicant: United States Surgical Corporation, Norwalk, Connecticut 06856 (US)
(72) Inventor: Totakura, Nagabhushanam, Norwalk, CT 06851 (US); Muth, Ross R., Brookfield, CT 06804 (US)
(74) Representative: Marsh, Roy David

(57) **Abstract**

A surgical bioabsorbable foam pledget is provided for use with a wound closure article such as a sutures or an annuloplasty ring. The surgical bioabsorbable foam pledget may optionally contain a medicinal agent.

## Description

### BACKGROUND OF THE INVENTION

### 1. Field of the Invention

The present invention relates to bioabsorbable foam pledgets for use in surgical closure of wounds.

### 2. Description of Related Art

Procedures for closing wounds are well known and numerous articles of manufacture have been developed to close wounds caused by surgery or trauma. Surgical sutures, staples, clips and clamps are frequently used to join damaged tissue together. However, in certain circumstances such wound closure articles may actually cut into and cause unintentional laceration of the tissue into which they are inserted. One technique used to prevent lacerations incorporates cushioning materials known as pledgets which act to buttress the tissue surrounding the area pierced by the wound closure article.

Ideally, pledgets should be manufactured from materials which are biocompatible, soft and durable. Examples of materials suggested for use in pledgets are disclosed in U.S. Patent Nos. 4,034,850 (polytetrafluoroethylene and polyester felt); 4,549,545 (continuous filament mat of a segmented urethane polymer); and European Patent Application No. 89100693.4 (polydioxanone, glycolide and lactide).

The above-noted materials used for pledgets may be associated with certain disadvantages. For example, as a result of inadvertent abrasion or decomposition, small portions of a polytetrafluoroethylene or urethane pledget may be released as debris or particles which can irritate surrounding tissue. If necessary, removal of pledgets made with these materials may be complicated by ingrowth of tissue into or around the pledget. Consequently, an improved pledget having excellent cushioning properties which avoids the above-noted disadvantages is desirable.

### SUMMARY OF THE INVENTION

The present invention provides bioabsorbable foam pledgets manufactured from homopolymers, copolymers, or block copolymers of hydroxyacids, carbonates, lactones, and oxalates or blends thereof for use in conjunction with the closing of wounds. Such foam pledgets may be combined with wound closure articles such as surgical sutures or annuloplasty rings.

### DETAILED DESCRIPTION OF THE INVENTION

Bioabsorbable foam pledgets according to the present invention are made from biocompatible hydrolyzable polymeric materials. Such materials include but are not limited to hydroxyacid derivatives such as glycolide, lactide, butyrates and valerates; carbonates such as trimethylene carbonate and hexamethylene carbonate; lactones such as caprolactone and dioxanone; and various combinations of these and related monomers. Polymers, copolymers, block copolymers, and blends of the afore-mentioned materials are known in the art and are disclosed, e.g., in U.S. Patent Nos. 2,668,162; 2,703,316; 2,758,987; 3,225,766; 3,297,033; 3,422,181; 3,531,561; 3,565,077; 3,565,869; 3,620,218; 3,626,948; 3,636,956; 3,736,646; 3,772,420; 3,773,919; 3,792,010; 3,797,499; 3,839,297; 3,867,190; 3,878,284; 3,982,543; 4,047,533; 4,060,089; 4,137,921; 4,157,437; 4,234,775; 4,237,920; 4,300,565; 4,523,591; 4,916,193; and 5,120,802; U.K. Patent No. 779,291; D.K. Gliding et al., "Biodegradable polymers for use in surgery-polyglycolic/poly(lactic acid) homo- and co-polymers": 1, Polymer, Volume 20, pages 1459-1464 (1979), and D.F. Williams (ed.), Biocompatibility of Clinical Implant Materials, Vol. II, ch. 9: "Biodegradable Polymers" (1981), which are hereby incorporated by reference. Preferred polymers for use in the present invention are glycolide, lactide, dioxanone and trimethylene carbonate.

In accordance with the present invention, a suitable bioabsorbable polymer is prepared and then made into a foam by methods such as those disclosed in U.S. Patent Nos. 3,902,497; 4,181,983; 4,331,652; 4,832,686; 4,960,866; 5,004,602; 5,102,983; United Kingdom Patent application GB 2,215,209; and European Patent Application No. 87311363-3. Indeed, any method of preparing a foam of any bioabsorbable polymeric material is contemplated by the present invention. See, e.g., European Patent Application No. 90300593.2 (alginate hydrogel foam) or European Patent Application No. 90314031.7 (biodisintegratable thermoplastic resin foam). The contents of the above noted documents are hereby incorporated by reference.

A preferred method of forming a bioabsorbable foam according to the present invention involves dissolving a bioabsorbable polymer in a suitable solvent, freeze drying the polymer solution and sublimation of the solvent. The solvent can be any solvent which is capable of dissolving the selected bioabsorbable polymer with the resulting polymer solution freezing solid when placed in a freezer or contacted with a freezing liquid which is immiscible with the solvent. More particularly, such solvents should possess a vapor pressure such that at or below the freezing point of the solvent, evaporation or sublimation can take place in vacuo. Suitable solvents include those selected from the group consisting of t-butanol, benzene, p-dioxane, p-xylene, 1,2-dibromoethane, morpholine, dimethylsulfoxide, bromoform, hexafluoroisopropanol (HFIP), hexafluoroacetone sesquihydrate (HFAS), and mixtures thereof, with p-dioxane and/or benzene being preferred.

The amount of polymer in the solution can vary from about 1% to about 30% by weight, the preferred concentration being about 15% by weight. After combining the polymer with solvent, the resulting solution is poured into a container. The thickness of the pledget can be varied depending on the height of the solution filled in the container.

The polymer solution is then frozen for about 48 to about 120 or more hours at temperatures of between about about -5_C and about -17_C. The frozen solution is freeze dried for about 72 to about 96 or more hours, i.e., until no more solvent is detectable in the foam. If residual solvent is found, it can be removed in situ. Alternatively, solvent may be removed by vacuum drying outside the container at a temperature of about 50_C.

The foam is then cut into a shape suitable for use as a pledget. For example, suitable shapes may be ellipsoidal, circular, rectangular, square, polygonal etc. Alternatively, bioabsorbable foam pledgets may be die-cast into the appropriate shape. The thickness of pledgets made in accordance with the present invention can range from about 0.5mm to about 2.0mm, but is preferably about 1.0mm.

Prior to use, a bioabsorbable foam pledget is threaded through or otherwise adhered to the wound closure article. For example, a suture may be threaded through the pledget and the pledget positioned at an appropriate point along the suture. Alternatively, bioabsorbable foam pledgets may be threaded through or adhered to the prong connecting bight of a surgical staple.

Bioabsorbable foam pledgets made in accordance with present invention are soft, resilient and demonstrate excellent stability in adhering to tissue. The foam construction of the pledget provides superior cushioning action between the wound closure article and underlying tissue. If particles of the bioabsorbable foam pledget are inadvertently separated from the pledget, unwanted side effects are minimized by degradation and bioabsorbtion of the particles. The size of pores contained in the foam can be made small or large to either avoid or allow ingrowth of tissue, respectively. The bioabsorbable nature of foam pledgets according to the present invention eliminates the need to remove the pledget after surgery. Moreover, the rate at which a bioabsorbable foam pledget is absorbed may be varied by changing the composition of the pledget, e.g., by incorporating more crystalline polymers which are more slowly absorbed than more amorphous polymers, and/or by increasing or decreasing the thickness of the pledget, and/or increasing or decreasing the void space contained within the bioabsorbable foam.

Pledgets made in accordance with the present invention may optionally include a medicinal agent incorporated into the polymer material or contained within the void areas of the foam. The term "medicinal agent" is used in its broadest sense herein and includes any substance or mixture of substances which are useful in medicine. Thus, it is understood that a medicinal agent may be a drug, enzyme, peptide, protein, dye, or diagnostic agent such as a releasable dye which may have no biological activity per se.

Examples of various classes of medicinal agents usable in accordance with the present invention include but are not limited to antimicrobials, analgesics, antipyretics, anesthetics, antiepileptics, antihistamines, anti-inflammatories, cardiovascular drugs, diagnostic agents, sympathomimetics, cholinomimetics, anti-muscarinics, antispasmodics, hormones, growth factors, muscle relaxants, adrenergic neuron blockers, anti-neoplastics, immunosuppressants, gastrointestinal drugs, diuretics, steroids and enzymes. It is also intended that combinations of medicinal agents can be used in accordance with the present invention.

The following examples help illustrate certain bioabsorbable foam pledgets according to the present invention. These examples should not be construed as limitations of materials or methods which may be used in accordance with the present invention.

### EXAMPLE 1

60.69 grams of lactide were copolymerized with 14.32 grams of p. dioxanone in the presence of 0.0150 grams of stannous octate at 150_C for 48 hours. A 15% by weight solution of the copolymer was prepared in p. dioxane solvent. Thus, 15 grams of the copolymer was dissolved in 100ml p. dioxane by stirring. The solution was allowed to settle for 30 minutes and then filtered to remove any suspended impurities. The filtered solution was allowed to settle for 30 minutes to remove any air bubbles.

The clear polymer solution was then poured into a petri dish or into a flat bottomed container to obtain a flat pledget material. The container or petri dish was covered with a glass cover mounted on thin shims placed on the sides of the petri dish containing the solution. The dish with cover containing the solution was then carefully placed in a freezer at about -17_C for about 120 hours. By this time, the polymer solution froze into a solid mass and the petri dish containing the frozen polymer was then transferred into a freeze drying apparatus. During the transfer necessary care was taken by keeping the container in a cooled beaker to avoid the melting of the frozen polymer. The frozen polymer was then freeze dried for about 72 to about 96 hours. The time for freeze drying may be adjusted to insure elimination of the solvent from resulting pledget material.

After freeze drying the polymer solution the pledget was removed and a portion of it tested for residual solvent by nmr spectroscopy. In cases where residual solvent was found freezed drying continued until residual solvent was removed.

### EXAMPLE 2

19.2 grams of lactide were copolymerized with 54.4 grams of trimethylene carbonate in the presence of 0.02 grams of stannous octate at 150_C for 24 hours. A 15% by weight solution of the copolymer was prepared in p. dioxane solvent. Thus, 15 grams of the copolymer were dissolved in 100ml p. dioxane by stirring. The solution was allowed to settle for 30 minutes and then filtered to remove any suspended impurities. The filtered solution was allowed to settle for 30 minutes to remove any air bubbles.

The clear polymer solution was then poured into a petri dish or into a flat bottomed container to obtain a flat pledget material. The container or petri dish was covered with a glass cover mounted on thin shims placed on the sides of the petri dish containing the solution. The dish with cover containing the solution was then carefully placed in a freezer at about -17_C for about 120 hours. By this time, the polymer solution froze into a solid mass and the petri dish containing the frozen polymer was then transferred into a freeze drying apparatus. During the transfer necessary care was taken by keeping the container in a cooled beaker to avoid the melting of the frozen polymer. The frozen polymer was then freeze dried for about 72 to about 96 hours.

After freeze drying the polymer solution the pledget was removed and a portion of it tested for solvent by nmr spectroscopy. In cases where residual solvent was found, freeze drying continued until residual solvent was removed.

### EXAMPLE 3

920 grams of glycolide were copolymerized with 5,203 grams of lactide and 5 grams of glycolic acid in the presence of 1.23 grams of stannous octate at 155_C for 15 hours. A 15% by weight solution of the copolymer was prepared in p. dioxane solvent. Thus, 15 grams of the copolymer were dissolved in 100ml p. dioxane by stirring. The solution was allowed to settle for 30 minutes and then filtered to remove any suspended impurities. The filtered solution was allowed to settle for 30 minutes to remove any air bubbles.

The clear polymer solution was then poured into a petri dish or into a flat bottomed container to obtain a flat pledget material. The container or petri dish was covered with a glass cover mounted on thin shims placed on the sides of the petri dish containing the solution. The dish with cover containing the solution was then carefully placed in a freezer at about -17_C for about 120 hours. By this time, the polymer solution froze into a solid mass and the petri dish containing the frozen polymer was then transferred into a freeze drying apparatus. During the transfer necessary care was taken by keeping the container in a cooled beaker to avoid the melting of the frozen polymer. The frozen polymer was then freeze dried for about 72 to about 96 hours.

After freeze drying the polymer solution the pledget was removed and a portion of it tested for solvent by nmr spectroscopy. In cases where residual solvent was found freeze drying was continued until solvent was removed.

The examples, embodiments and methods depicted in the specification are not intended to be limitations of the inventive concept described herein. Accordingly, modifications may be made which are intended to be within the scope of the following claims.

## Claims

1. A surgical article for use in conjunction with surgical wound closure comprising a pledget comprising bioabsorbable foam polymeric material.

2. A surgical article according to claim 1 wherein the bioabsorbable foam polymeric material is selected from the group consisting of homopolymers, copolymers or block copolymers of hydroxyacids, carbonates, lactones and oxalates.

3. A surgical article according to claim 2 wherein the bioabsorbable foam polymeric material is selected from the group consisting of glycolide, lactide, dioxanone and trimethylene carbonate.

4. A wound closure article/pledget combination comprising a wound closure article in contact with a pledget according to claim 1.

5. A wound closure article/pledget combination according to claim 4 wherein the wound closure article is selected from the group of a suture and an annuloplasty ring.

6. A surgical article according to claim 1 further comprising a medicinal agent.

7. A process for manufacturing a bioabsorbable foam pledget comprising:
(i) providing a bioabsorbable polymeric material;
(ii) dissolving the bioabsorbable polymeric material in a solvent and forming a polymer solution;
(iii) removing the solvent from the polymer solution;
(iv) forming a bioabsorbable polymeric foam; and
(v) shaping the bioabsorbable polymeric foam into a pledget.

8. A process for manufacturing a bioabsorbable foam pledget according to claim 12 wherein the bioabsorbable polymeric foam is formed by:
(i) freezing the polymer solution;
(ii) placing the polymer solution in a vacuum; and
(iii) removing the solvent.

9. A bioabsorbable foam pledget manufactured by a process according to claim 12.
